# EUROPEAN PATENT SPECIFICATION

(11) **EP 1 730 265 B1**
(45) Date of publication and mention of the grant of the patent: **17.02.2016**
(21) Application number: 05726955.7
(22) Date of filing: 23.03.2005
(51) Int. Cl.: A61K 35/12

(54) **3D-CARDIAC TISSUE ENGINEERING FOR THE CELL THERAPY OF HEART FAILURE**
3D-GEWEBETECHNOLOGIE FÜR DIE ZELLTHERAPIE BEI HERZVERSAGEN
MODIFICATION GENETIQUE DE TISSU CARDIAQUE EN TROIS DIMENSIONS POUR LA THERAPIE CELLULAIRE D'INSUFFISANCE CARDIAQUE

(30) Priority: 24.03.2004 US 555815 P
(43) Date of publication of application: 13.12.2006
(73) Proprietor: Jaconi, Marisa, E., E., 1203 Geneva (CH); Zammaretti-Schaer, Prisca, 6330 Cham (CH)
(72) Inventor: Jaconi, Marisa, E., E., 1203 Geneva (CH); Zammaretti-Schaer, Prisca, 6330 Cham (CH)
(74) Representative: Sarpi, Maurizio
(86) International application number: PCT/IB2005/000752
(87) International publication number: WO 2005/093047

(56) References cited:
- WO-A-03/020920
- WO-A-03/085092
- WO-A-2004/037188
- WO-A-2005/010172
- WO-A1-00/61204
- DE-A1- 10 158 680
- US-A1- 2002 146 401
- MANN BK AND WEST JL: "Tissue Engineering in the Cardiovascular System: Progress Toward a Tissue Engineered Heart" THE ANATOMICAL RECORD, vol. 263, 2001, pages 367-371, XP002356733
- NAGATA M ET AL: "Efficient gene transfer of a simian immuno-deficiency viral vector into cardiomyocytes derived from primate embryonic stem cells" JOURNAL OF GENE MEDICINE, WILEY,, US, vol. 5, no. 11, November 2003 (2003-11), pages 921-928, XP002279843 ISSN: 1099-498X
- LIU J ET AL: "Autologous stem cell transplantation for myocardial repair" AM J PHYSIOL HEART CIRC PHYSIOL, vol. 287, 2004, pages H501-H511, XP002356304
- LU, LI ET AL: "Molecular and cellular events at the site of myocardial infarction: from the perspective of rebuilding myocadial tissue" BIOCHEMICAL AND BIOPHYSIAL RESEARCH COMMUNICATION, vol. 320, 2004, pages 907-913, XP002356305
- SOUKIASIAN HJ ET AL: "A Novel Sub-Population of Bone Marrow-Derived Myocardial Stem Cells: Potential Autologous Cell Therapy in Myocardial Infarction" J HEART LUNG TRANSPLANT, vol. 23, 2004, pages 873-880, XP002356306
- SAM J ET AL: "Heart cell implantation after myocardial infarction" CORONARY ARTERY DISEASE, vol. 16, no. 2, March 2005 (2005-03), pages 85-91, XP009056054

## Description

The present invention relates to the field of tissue engineering and in particular to the use of stem cells compositions for treating heart failure, wherein said compositions do not comprise human embryonic stem cells.

### Background of the invention

New steps in regenerative medicine are showing the first advances in the application of stem cells for tissue regeneration. Up to now, cardiac tissue engineering utilizes cells seeded into polymeric scaffolds to try to reproduce the myocardial structure and properties. However, among other problems, neovascularization is still a limiting factor while using conventional tissue scaffolds.

Cell therapy may be successfully used for the treatment of heart failure caused by myocardial infarction, thanks to a multidisciplinary approach combining expertise in stem cells biology, tissue engineering as well as non-invasive cardiac imaging.

Over the past few years, research on animal and human stem cells (either embryonic, fetal or adult stem cells) has experienced tremendous advances which are almost daily loudly revealed to the public on the front-page of newspapers. The reason for such an enthusiasm over stem cells is that they could be used for the treatment of spontaneous or injuries-related diseases that are due to particular types of cells functioning incorrectly, such as cardiomyopathy, diabetes mellitus, osteoporosis, cancers, Parkinson's disease, spinal cord injuries or genetic abnormalities. This new concept of "regenerative medicine" by stem cell therapies is unprecedented since it involves the regeneration of normal cells, tissues and organs. Embryonic stem cells present at the blastocyst stage are defined as pluripotent since they have the tremendous ability to self-renew and also to differentiate into a variety of cells of all 3 germ layers. Adult stem cells present in some organs also have some regenerative capacities (i.e. satellite cells and bone marrow cells). However their potential to transdifferentiate to phenotypes different from those to which they are pre-committed seems, so far, more restricted. For this reason, they are defined as multipotent. On the other hand, these cells offer the advantage to be used in autologous cell transplantation, avoiding problems of rejection and immunosuppressive drugs. Therefore, research on both type of stem cells is complementary and needs to be pursued in parallel.

Heart failure is the number one cause of death in industrialized countries. Myocardial infarction typically results in fibrotic scar formation and permanently impaired cardiac function because, after a massive cell loss due to ischemia, the myocardial tissue lacks intrinsic regenerative capability. Thus, efforts to regenerate functional myocardial tissue are being firstly pursued through cell grafting. The principal feasibility of using cardiac cells for heart tissue regeneration has been achieved nearly 10 years ago. Thereafter, several groups have enlarged our knowledge about the fate of implanted cells of various origin (embryonic, fetal or adult) in the myocardium of healthy and diseased heart. Most studies support the notion that cell engraftment in models of myocardial infarction can improve contractile function. There are presently several ongoing clinical studies using adult stem cells (skeletal myoblasts, bone marrow stem cells) to investigate the safety and feasibility of such a cardiac cell therapy. However, there is still no convincing demonstration for a transdifferentiation to the cardiac phenotype of such adult stem cells. So far, first clinical results are still controversial and demonstrated the need to better understand stem cell biology.

Cardiac tissue engineering includes the fields of material sciences and cell biology. It has emerged as an alternative promising approach to replace or support an infarcted cardiac tissue and thus may hold a great potential. Tissue engineering involves the construction of tissue equivalents from donor cells seeded within 3D biomaterials, then culturing and implanting the cell-seeded scaffolds to induce and direct the growth of new, healthy tissue. Tissue-engineering technologies have therefore the potential to revolutionize soft-tissue reconstruction by creating biologically-based tissue replacements.

After myocardial infarction, the lost cardiomyocytes due to a lack of vascularization are replaced by a scar tissue. The surviving cardiac cells undergo neurohormonal-induced hypertrophy as a compensatory mechanism in order to maintain a demanded cardiac output. With time, the heart wall becomes thinner and the ventricle dilates, leading to an end-stage congestive heart failure. Therefore, the possibility to repopulate these infarcted areas would be essential for the restoration of a functional contracting myocardium. Repopulation of such tissues by the application of extracellular matrix-based gels engineered with stem cells is the goal. The plasticity of the stem cells and the presence of appropriate growth factors and cytokines will enable to guide the differentiation of stem cells into functional cardiomyocytes. Furthermore, the addition of other growth factors and bioactive molecules will enable us to provide the region with an enhanced vascularization (also via the incorporation of endothelial progenitor cells), necessary for the viability of the patch and the integration into the myocardium.

Cardiac tissue engineering methods typically utilize cells seeded into or onto polymeric scaffolds. Reproducing the special organizational, mechanical, and elastic properties of native myocardium represents a significant challenge from the perspective of tissue engineering scaffolds. Ideally, these constructs display properties of native myocardium such as coherent contractions, low diastolic tension, and syncytial propagation of action potentials. Engineered tissue constructs should have the propensity to integrate and remain contractile *in vivo.* In order to better meet the mechanical demands of force-generating contractile tissue, biodegradable materials such as hydrogels may serve as more appropriate scaffolds.

Recent attempts to engineer soft tissue include the use of scaffolds manufactured from natural polymers, such as collagen, gelatin, alginate hyaluronic acid and chitosan gels or synthetic, biodegradable polymers.

So far, various methods to produce 3D-cardiac tissue constructs have been proposed, but the reconstruction of a functional heart tissue remains to be achieved. In particular, problems with vascularization still limit the use of conventional tissue scaffolds in the replacement of large-size tissue defects.

Bioactive materials are materials which incorporate growth factors or cytokines and are able to release them either at a controlled ratio or directly on cell demand. Extracellular matrix (ECM) components like fibrin or collagen, or synthetic polymers mimicking the ECM, like the polyethylenglycol-modified polymers, can be used as vehicles. These materials offer the cells an environment providing adhesion natural sites, eventually growth factors and cleavage sites enabling the cells to substitute the material while proliferating and migrating.

Native ECMs components such as fibrinogen from human plasma are particularly useful since it can be cleaved by thrombin into its fibrin subunits. After cleavage a self-assembly step occurs in which the fibrinogen monomer come together and form a non-covalently cross-linked gel via proteolytic exposure of binding sites. The covalent crosslinking of the chains is achieved via factor XIIIa, which is a trasglutaminase. Through factor XIIIa it was shown that other proteins like fibronectin or collagen can be bound.

By protein-engineering it is possible to produce growth factors and cytokines, which have an alpha2-plasmin inhibitor sequence in their N-terminus. Gels containing such architectures, can trap growth factors and cytokines, which can be subsequently cleaved on cell demand. Therefore, the degradation ratio of the gel as well as the release of growth factors will be cell proliferation dependent.

Cardiomyocytes are the main cellular component of the heart, but non-muscle cells (such as endothelial cells, mesenchymal cells, fibroblasts, smooth muscle cells and leukocytes) play an important role in cardiac development and function. The exact contribution of each single cell type to tissue-formation has not been deeply analyzed yet, but the presently available data strongly suggest that formation of a true cardiac tissue-like 3D construct requires the presence of cardiac myocytes and non-myocytes in physiological mix. Embryonic stem cells (ESC) are pluripotent cells derived from the inner cell mass of the blastocyst. Therefore they have the ability to differentiate into many kind of cells, a capacity that becomes progressively restricted with development. Unlimited differentiation capacity and indefinite propagation represent the strongest advantages of ESC.

Pluripotent ESC are able to differentiate *in vitro* into structures called embryoid bodies (EBs). During this differentiation process, ESCs develop into a variety of committed cell lineages originating from mesoderm, ectoderm and endoderm. Therefore, the possibility to isolate, from a variety of differentiating cells, embryonic cardiomyocytes that are still in a proliferative phase offers a tremendous advantage. Several studies have demonstrated the possibility to engraft such cells into animal models of heart failure with encouraging results. Since the derivation in 1998 of human ESCs lines from pre-implantation embryos, considerable research is centered on their biology, in particular on how to encourage a specific cell differentiation and also on means to enrich and purify derivative cell types such as cardiomyocytes.

Cardiac differentiation mechanisms have been intensively studied in several models both *in vivo* (by trangenesis or gene-deletion studies in mice) and *in vitro* using strategies to enhance cardiogenesis in cultured ESC. These have allowed the identification of important growth factors, morphogens, as well as transcription factors regulating cardiogenesis.

Heart formation is induced within the anterior mesoderm and results from a complex interplay of growth factors and morphogens. The role of the endoderm in releasing pro-cardiogenic factors has been demonstrated in several species. For example, in the presence of visceral endoderm-like cell line END2, cardiac differentiation was induced in mouse ESC without the need of EB formation providing cells displaying nodal-like, atrial-like and ventricular action potentials. Factors like Activin A and TGFβ1 are capable to turn on the expression of mesodermal genes. An other important factor seems to be Wnt11. The Wnt family of secreted glycoproteins has been involved in several morphogenetic and differentiation processes. The family is divided in 2 classes depending on which signaling pathway they channel. Wnt1/-8/-3 activate the canonical Wnt/β-catenin pathway. This pathway has to be actively inhibited for cardiogenesis to occur. On the other hand, other members of the family such as Wnt11/-4/-5a activate the non-canonical Wnt/Ca²⁺ or Wnt/JNK pathways, which actively promote cardiogenesis. The human Wnt11 gene, cloned in 2001, is expressed in fetal lung, kidney, adult heart, liver, skeletal muscle, and pancreas, as well as in several human tumors. In the quail, Wnt11 could induce cardiogenesis from non-cardiogenic mesoderm explants. In vertebrates, studies on mouse embryonic carcinoma cells showed that Wnt11-conditioned medium could trigger cardiac differentiation. In Xenopus, high doses of Wnt11 induced expression of cardiac markers such as Nkx2.5 and MHCα and beating tissue was produced. Recently, Terami *et al.* showed that treatment of mouse ESC with Wnt11 could increase the expression of cardiac markers as well as the expression of GFP knocked in the Nkx2.5 locus. Interestingly, other cells such as circulating endothelial progenitor cells, when cocultured with neonatal cardiomyocytes, were also shown to differentiate more efficiently towards cardiac-like cells in the presence of Wnt11-conditionned medium. Growth factors and morphogens act through activation of signaling cascades that lead to expression of specific transcription factors. Several families of transcription factors have been implicated in cardiac specification and differentiation: Nkx-type, MEF-type, Tbx-type and GATA-type. Nkx-type factors are a family of homeodomain transcription factors and, in particular, one of its members, Nkx2.5 (named CSX in humans) is crucially implicated in cardiogenesis, particularly at the moment of mesoderm specification. Being expressed very early in the mouse, Nkx2.5 deletion is embryonic lethal due to a disrupted looping process. Nkx2.5 has been shown to interact with several other transcription factors as well as to directly induce the expression of many cardiac structural proteins.

MEF2C is a member of the MADS-box family of transcription factors, which is expressed at very early times in the developing heart. In mice, homozygous null mutations result in abnormal looping, absence of right ventricle and absence of expression of a subset of cardiac genes. Its effect is mediated through interaction with other cardiac transcription factors such as GATA factors, Nkx2.5 and dHAND. In our previous work, we have shown that Ca²⁺ signaling disturbance provoked by the absence of calreticulin in mouse ESC ultimately prevents MEF2C phosphorylation and thus translocation into the nucleus, inhibiting the expression of cardiac genes such as MLC2v.

Published studies have so far focused on spontaneous cardiac differentiation of hESC within embryoid bodies. The effect of overexpression or ablation of specific transcription factors still need to be addressed in differentiating hESC.

WO03/020920 discloses an improved method for culturing human pluripotent stem cells. According to this invention the pluripotent stem cells are cultured in presence of a component added to the medium in order to support rapid cell proliferation without differentiation.

The US Patent. Application published US 2006/0051330 discloses a two-stage method for carrying out the expansion and differentiation of multipotent stem cell *ex vivo.* In the first stage, during the expansion, the stem cells can be gene transfected; in the second stage, the differentiation of the multipotent stem cells takes place optionally into cells of the hematopoietic, endothelial or mesenchymal cell lineage which can be used for the prophylaxis, diagnosis and therapy of human disease and for tissue engineering. The US Patent Application published US 2002/0146401 discloses methods of forming and using signaling complexes expressed in specific tissues and organs during particular stages of development to predicably guide cell division, cell migration, differentiation of cells and/or formation of tissue or organ primordia *in vitro.*

WO03/085092 discloses the method to obtain myoblast cells by culturing satellite and progenitor cells to be transplanted into tissue such as diseased heart tissue to form healthy repair tissue and reverse disease. Brenda K. Mann and Jennifer L. West in "Tissue Engineering in the Cardiovascular System: Progress Toward a Tissue Engineered Heart", The Anatomical Record, 2001, 263: 367-371, describe tissue engineering techniques which may be used within the cardiovascular system to improve the function of the native tissue, for instance for treatment of restenosis or congestive heart failure.

Mihoko Nagata et al. in "Efficient gene transfer of a simian immunodeficiency viral vector into cardiomyocytes derived from primate embryonic stem cells", The Journal of Gene Medicine, 2003, 5: 921-928, have evaluated the survival of transplanted cardiomyocytes derived from cynomolgus monkey (*Macaca fascicularis*) ES cells in the injured myocardium of a rat myocardial infarction model.

WO 2005/010172 describes a quite complex semi-solid 3D biocompatible scaffold for cell growth, tissue repair and regeneration of the heart, comprising two or more scaffold layers of alternating attachment (A)-strips (composed of extracellular matrix material of natural origin, i.e. from tissues such as submucosa or synthetic, such as PLA, PGA, etc.), and separating (S)-strips together with one or more separating layers, composed of cross-linked alginate. According to the invention the A-strips are intended to provide spatial and/or orientation-dependent signals thereby inducing ordered alignment of the cells. S-strips and separating layers are intended not to favour cell adhesion. Fibrin is included as a possible component of S-strips and not as a cell seeding matrix.

WO 2004/037188 discloses a method for producing cardiomyocytes in vivo by administering to the heart of an individual a cardiomyocytes producing amount of mesenchymal stem cells. These cells can be administered as an injectable liquid or as a cell preparation in a matrix which is, or becomes, solid or semi-solid and can be genetically modified to enhance myocardial differentiation and integration.

Soukiasian et al. in "A novel sub-population of bone marrow-derived myocardial stem cells: potential autologous cell therapy in myocardial infarction" in J. Heart Lung Transplant, 2004, 23: 873-880, have analyzed the ability of bone marrow-derived beta2-microglobulin-negative cells to differentiate into cardiomyocytes and reconstitute the myocardium in a model of myocardial infarction.

WO0061204 discloses a method promoting blood vessel formation in tissues and organs enabling the implantation or attachment of a 3D-stromal tissue to promote endothelialisation and angiogenesis in the heart and related tissue; the method provide tissue repair and regeneration and uses fibrin as a glue sealant to attach the 3D-stromal tissue or scaffold to the heart surface.

### Detailed description of the invention

In view of the prior art, new cardiac patches have been developed in order to overcome the previously mentioned technical problems. In particular, extracellular matrix-based gels containing stem cells excluding human embryonic stem cells have been designed as cell therapy strategies to renew infarcted tissues and thereby treat heart failure. In addition, the use of bioactive and biodegradable materials, able to locally release appropriate growth factors, cytokines and bioactive molecules into the material in which stem cells are seeded, lead to the improvement of differentiation of non human embryonic stem cells (ESC) into viable cardiomyocytes and allow endothelial progenitor cells (EPCs) to build a vascular bed. Moreover, used cells can be appropriately genetic-engineered in order to facilitate selection and further differentiation.

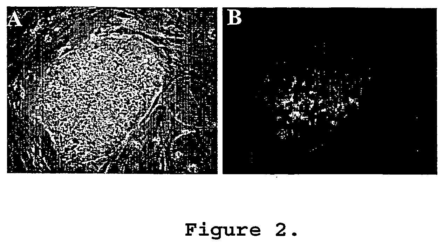

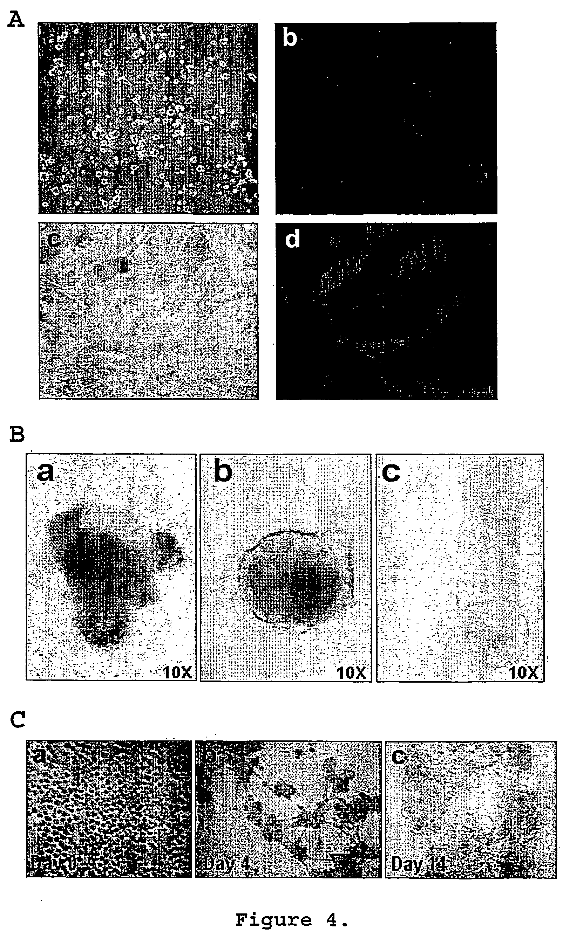

The advantages and features of the present invention will be clarified in the following detailed description together with preferred embodiments shown in the examples with reference to the attached figures wherein:

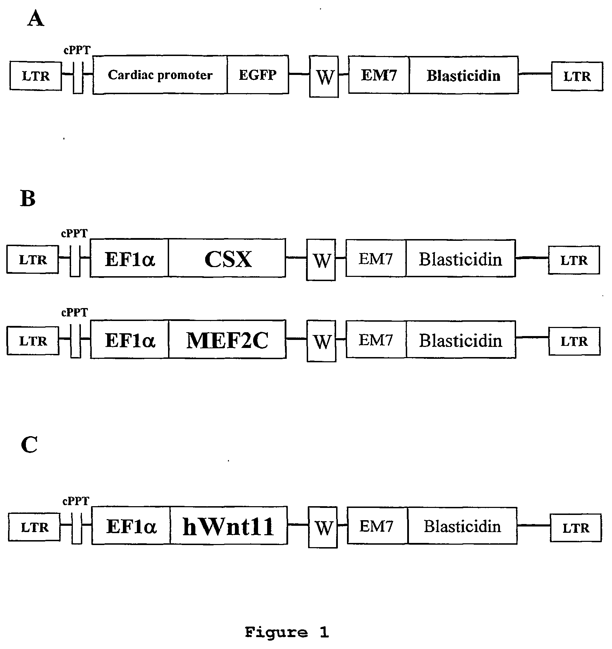

Figure 1 shows lentiviral vectors modified from pLenti6/BLOCK-iT-DEST. Vector in A comprises cPPT= central polypurine tract cassette, cardiac-specific promoter inserted in a multiple cloning site, EGFP gene, w= woodchuck cassette, EM7 constitutive promoter, blasticidin resistance gene. Vector in B enables the stable overexpression of cardiac transcription factors. The promoter driving EGFP can be a constitutive (EF1α) or cardiac specific. Vector in C is for the expression of human Wnt11 gene.
Reference Figure 2 shows transduction of hESC with a gateway lentiviral vector containing EGFP under the control of the EF1α promoter. A) phase contrast and B) fluorescence imaging (FITC) of a colony containing transduced EGFP-positive undifferentiated hESC.

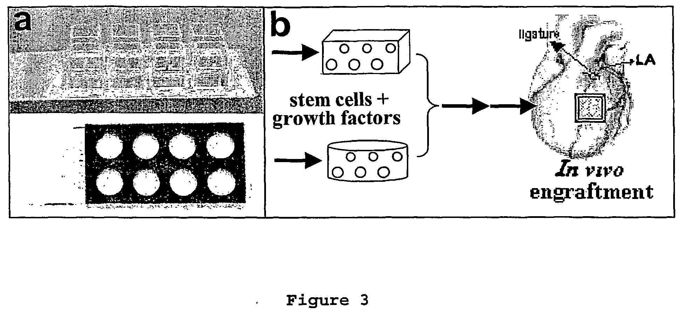

Figure 3 Shows the scheme of the *vitro* preparation of 3D-tissue engineered fibrin gels. In (a) different multi-well systems are used to produced 3D fibrin gels containing appropriate types of stem cells and growth factors to study their differentiation and tissue formation *in vitro.* In (b) such engineered "cardiac patches" are then implanted onto normal or infarcted rat hearts.
Reference Figure 4A shows ESC stable clones containing the CD63-GFP marker gene incorporated into 3D-fibrin gels and observed at day 0 (a,b) and day 4 of culture (c,d). Figure 4B shows differentiation of mouse embryoid bodies (EBs) into contracting cardiomyocytes. Day 6-EBs were either adhered on gelatin-coated plates (A), on top of fibrin gels(B), or within 3D fibrin gels (C). After 2 days of culture (Day 8), clusters of contracting cardiomyocytes were observed (dashed circles) showing that differentiation can proceed normally in all culture conditions. Figure 4C illustrates that freshly-dissociated and purified neonatal rat cardiomyocytes are able to redifferentiate within the 3D gels, forming a long-lasting network of spontaneously beating cells (a-c).

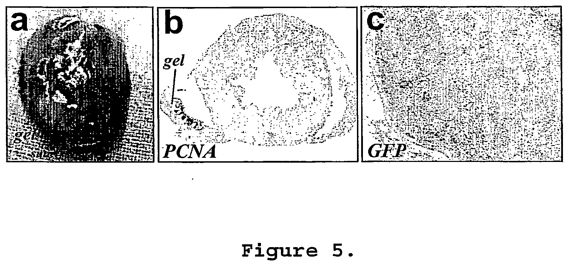

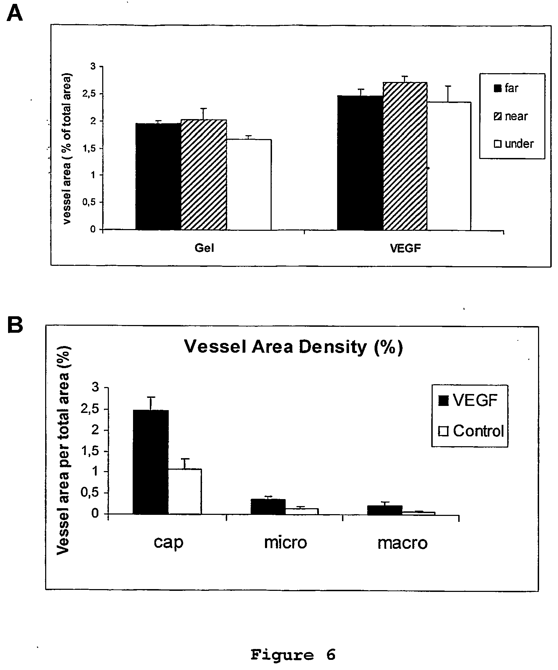

Figure 5 shows ex-vivo rat heart (a) engrafted with a 3D-fibrin gel containing undifferentiated CD63-GFP-labeled ESC for a period of 2 weeks. After 2 weeks, immunohistochemistry staining revealed a gel full of proliferating ESC, positive for the PCNA (proliferating cell nuclear antigen) (b), and the GFP marker (c). Figure 6 shows the neoangiogenic effect of a fibrin gel containing VEGF applied to a normal (A) or an infarcted (B)rat heart for 8 weeks. Vessels (capillaries, micro-and macro-vessels) were counted using a computer program.

### Genetic-engineered ESC for cardiac cell selection

The stable expression of a transgene is a crucial tool to study cell specification and modulate the differentiation potential of hESC, not part of the invention. Morevoer, this strategy enables the selection of specific cell types using reporter or selection genes driven by specific promoters. We have indeed generated hESC-transduced lines, which are not part of the invention, using lentiviral vectors (Fig. 1) carrying reporter and selection genes, enabling to obtain pure populations of transduced hESC after selection using a marker gene and/or an antibiotic resistance. It also enables to identify transduced cells when exposed to other cell types (in coculture experiments). To select transduced hESC, not part of the invention, using an antibiotic resistance, we first established suited conditions to grow hESC on irradiated STO feeder cells, previously engineered to express different antibiotic cassettes, such as blasticidin and neomycin.

Human ESC, not part of the invention, are efficiently transduced with a lentiviral vector expressing GFP under the control of the EF1α promoter. We showed that EGFP transduction of hESC colonies in suspension was more effective than transduction of attached cells, obtaining as much as 70% GFP positive cells without cell sorting, as shown in figure 2.

To identify, quantify as well as select cardiogenic cells during different differentiation protocols we generate stable hESC lines, which are not part of the invention, transduced with lentiviral vectors enabling the expression of EGFP driven by cardiac specific promoters. We use Gateway lentiviral vectors allowing to recombine a promoter of interest and a gene of interest in a destination vector (modified from pLenti6/BLOCK-iT-DEST, Invitrogen). To ensure an optimal expression level, 2 supplementary cassettes (cPPT, central polypurine tract and W, Woodchuk) have been inserted. Such a lentiviral vector enables us to choose both the promoter and the gene of interest for the selection of specific cardiac cell populations. Cardiac promoters comprise the 450 pb α-cardiac actin promoter enabling the selection of all the cardiac cells, and the α-MHC promoter, shown to preferentially label pacemaker and atrial cells in mouse ES cells. Transduced cells are selected either by blasticidin resistance or FACS-sorted.

### Constitutive or inducible expression of early cardiac transcription factors: CSX and MEF2C

CSX and MEF2C are two of the earliest known transcription factors expressed in cardiogenic precursor cells. We are assessing the effect of a stable CSX overexpression on cardiac differentiation. To this purpose we have cloned the human CSX from a human heart RNA (Ambion) and recombined it in our modified gateway lentiviral vector containing the Blasticidin resistance as shown in Figure 1. Untransduced cells are eliminated by blasticidin treatment and stable clones are selected and maintained and propagated in culture. As the CSX vector and vector with the α-cardiac actin promoter driving the EGFP contain 2 different antibiotic resistance cassettes (neomycin and blasticidin), we can generate double-transduced cell lines, carrying GFP plus the gene of interest. The same strategy is applied for the expression of MEF2C.

### Exposure to growth factors/signaling molecules derived from endodermal cell derivatives

Embryonic and extra-embryonic tissues are supposed to contribute to cardiac lineage commitment before and during gastrulation in a paracrine fashion. Evidence has accumulated that factors secreted by the anterior lateral endoderm and extra-embryonic endoderm contribute to cardiomyogenesis. As well known, visceral-endoderm (VE)-like cell lines (P19-derived END-2 cells) induce mouse and human embryonic stem (ES) cells to aggregate spontaneously in coculture and differentiate to cultures containing beating muscle.

In line with this, our strategy is to enhance cardiac specification and differentiation by exposing hESC to conditioned medium derived from murine teratocarcinoma F9 cells. Indeed, upon treatment with retinoic acid (RA) and dibutyryl cyclic AMP (Bt₂cAMP), F9 cells differentiate into parietal endoderm-like cells, reduce their proliferation rate and express basement membrane components including laminin -1 subunits (α1, β1, and γ1), collagen IV subunits (α1 and α2), and heparan sulfate proteoglycan core proteins (including perlecan, present in the basal surface of myocardium and endocardium during development).

Conditioned medium from RA-differentiated F9 cells is collected and used to stimulate hESC during their early differentiation into EBs (from day 0 to 2, from day 3 to 5, and from day 6 to 10). The first human beating cardiomyocytes are usually observed from day 12 on. In mouse these are observed already at day 7-8 of culture.

### Differentiation of ESC in 3 dimentions using 3D fibrin-based biomatrices

The aim of the invention is to treat heart failure by controlling and enhancing cardiac differentiation of non human embryonic stem cells incorporated into 3D biomatrices composed of bioactive resorbable materials able to incorporate growth factors, cytokines, peptides, bioactive molecules and relevant proteins of the extracellular matrix (ECM).

In a first stage, differentiation of mouse ESC is performed principally using fibrin-based 3D matrices which are particularly interesting due to their easy availability, biodegradability and convenience. They offer in addition the advantage to insert growth factors that can freely diffuse in the matrix.

Our preliminary experiments have established the feasibility of growing and differentiating embryonic stem cells in 3D matrices as shown in figures 3 AND 4A. Also differentiating mouse EBs can migrate and colonize the fibrin gels and are able to show areas of contractile cardiomyocytes beating in 3 dimensions, as shown in figure 4B. Both isolated neonatal and ES-derived cardiomyocytes cultured whithin 3D fibrin gels are also able to reaggregate in bundles while retaining their beating activity, as shown in figure 4C.

This type of strategy, allow us to: 1) define the optimal conditions to seed the non human ESC or the selected non human ESC-derived cardiomyocytes onto fibrin gels at a high and spatially uniform initial density in order to maintain their viability and function; 2) incorporate specific growth factors and/or cytokines as well as ECM components (such as laminin) in order to support and guide the tissue formation from dissociated cells as a function of time. These factors can be freely trapped into the gel mesh. Some factors could also be covalently conjugated to fibrin and will then be available to the cells upon fibrin degradation by the cells, via the release of metalloproteinases. The factors of interst comprise Wnt11, transforming growth factor beta (TGFβ), insulin growth factor 1 (IGF-1), VEGF (vascular endothelial growth factor), epidermal growth factor (EGF), plateled-derived growth factor (PDGF), placental growth factor (PLGF), keratinocyte-derived growth factor (KDGF).

Cytokines of interest comprise the interleukin 6 (IL-6) family of cytokines, including IL-6, leukemia inhibitory factor (LIF), soluble kit ligand (sKitL) and cardiotrophin-1. They have a variety of biological functions, including on cardiovascular systems. The IL-6 family regulates growth and differentiation of many types of cells. LIF has been reported to ameliorate denervation-induced muscle atrophy and improve regeneration of muscle and nerves. In the heart, gp130, the common receptor of the IL-6 family, is expressed abundantly and has been reported to be critically involved in the growth and survival of cardiomyocytes. It has also been reported that LIF receptor is expressed abundantly in cardiomyocytes and that LIF induces marked cardiomyocyte hypertrophy and promotes survival of cardiomyocytes.

Other molecules of interest for cell and heart function may also be included in the 3D matrices before implantation. These may comprise ß-blockers, known to exert antiarrhythmic effects which are particularly important since altered calcium handling makes failing hearts very susceptible to arrhythmias. Also, ß-blockers can prevent the hypertrophic, proapoptotic, and pronecrotic effects of cardiomyocyte ß₁-receptor stimulation. In addition, ß-blockers can improve the energy balance in failing hearts by reducing heart rate and improve diastolic filling and blood flow. Moreover, ß-blockers reverse failure-specific alterations in cardiac gene expression, which may be involved in progression of the disease. The local infusion of these molecules upon matrix implantation may also have local beneficial effects on electrical properties of cells in the myocardium.

### Non-genetic isolation of cardiogenic stem cells

The method is defined to separate cardiac cells by their density using finely-tuned Percoll gradients, taking advantage on the enrichment in muscle contractile proteins within differentiating cardiac cells. Two different types of discontinous Percoll gradients allows 1) the isolation of early cardiogenic cells at day 5-6 from mouse ESC differentiating into embryoid bodies (gradient 1), and 2) the isolation of ES-derived differentiated cardiomyocyes at day 10-12 of culture (gradient 2).

After enzymatic dissociation (using 50ml ADS buffer containing 30 mg collagenase CLSII and 5 mg pancreatin), cells are separated by 30 min centrifugation (at 3000 rpm) on a Percoll gradients prepared from a Percoll stock solution (d= 1.11, mix 45 ml Percoll + 5 ml ADS 10X). The gradient 1 is composed as follow: 3 ml of a bottom layer (D1= 1.09 : 7.5 ml Percoll stock + 2.5 ADS 1X), and 4 ml of a top layer (D2=1.07 : 5.5 ml Percoll stock + 5 ml ADS 1X). Gradient 2 is composed as the previous one except that the D2 is = 1.05).

ADS buffer contains (in gr/lt) NaCl 6.8; HEPES 4.76; NaH₂P0₄ 0.12; Glucose 1.0; KCl 0.4; MgSO₄ 0.1.

### Stimulation of cell migration in 3D

We designed a strategy to favor cell migration within the 3D gels. To this purpose we took advantage of the properties of thymosin β4, a 43-amino-acid peptide expressed in several tissues including the developing heart. Thymosin β4 is present in very high concentrations in white blood cells and is released if clotting occurs.

The most prominent function of thymosin β4 is the sequestration of G-actin monomers, thus affecting actin-cytoskeletal organization necessary for cell motility. Thymosin β4 may also affect transcriptional events by influencing Rho-dependent gene expression events regulated by nuclear actin. Interestingly, thymosin β4 was reported to stimulate migration of cardiomyocytes and endothelial cells and to promote survival of cardiomyocytes. Treatment of mice with thymosin β4 after coronary ligation resulted in increased phosphorylation of the survival Akt in the heart, enhanced early myocyte survival and improved cardiac fuction.

Thymosin β4 can be incorporated into fibrin and fibrinogen by covalently cross-linking with factor XIII. The incorporation site localizes within the Aα392-610 region of the fibrin(ogen) αC-domains. Thymosin β4 is therefore incorporated into the fibrin gels in order to support cell survival and favor cell migration into the tissue in need of cell regeneration. Another factor enhancing cell motility/migration in the case of hematopoietic stem cells is the soluble kit ligand (sKitL, see next paragraph on EPC).

### EPCs-dependent vascularization

Endothelial progenitor cells (EPCs) isolated from bone marrow, peripheral or cord blood play a crucial role in many physiological and pathological situations. These cells participate to complex processes like angiogenesis and arteriogenesis, which are important steps in vascular repair. The involvement of these cells in the neovascularization of tissues was observed in animal models during tumor development as well as during therapeutic angiogenic assays. These cells have shown promising results when transplanted in ischemic limb and into the heart.

EPCs are known for their high proliferation rate and capability to support angiogenesis and revascularization in ischemic tissues and differentiate into the terminal phenotype of endothelial cells (ECs). Their proliferative potential, when compared to the one of endothelial cells, as well as their differentiation pathways into endothelial cells, smooth muscle cells and cardiomyocytes, makes them suitable candidates.

To completely restore cardiac function in infarcted areas, however, EPCs are not sufficient, in fact only in the presence of cardiomyocytes, this transdifferentiation is possible.

Growth factors like VEGF are needed in an appropriate local concentration to induce angiogenesis but avoid hemangioma formation. Pericyte recruitment is necessary to capillary formation and maintenance. Like the angiogenic response of endothelium to VEGF, pericyte recruitment is a process that is highly dependent on plateled-derived growth factor-B (PDGF-B) microenvironmental distribution.

Exogenous administration of angiopoietin-1 has been shown to stabilize new vessels and counteract VEGF-induced vascular leakage and edema. It is therefore a candidate for co-delivery with VEGF. Similarly, PDGF-B could modulate VEGF effect and prevent deleterious effect of prolonged high doses of VEGF.

Matrix metalloproteinases (MMPs)are able to promote the release of extracellular matrix-bound or cell-surface-bound cytokines, such as VEGF, which then can regulate angiogenesis. Accordingly, MMPs may contribute to the release of stem cell-active cytokines that stimulate stem cell proliferation. In particular, MMP-9 rapidly releases the stem cell-active cytokine sKitL (s: soluble form). Increased bioactive sKitL promotes hematopoitetic stem cell cycling and enhances their motility, both of which are essential cell survival, proliferation and differentiation.

The effect of fibrin gels containing VEGF applied to a normal or an infarcted rat ventricular myocardium has been investigated. Figure 6 illustrates that, both in normal and infarcted hearts, the cardiac tissue in contact with the VEGF-matrix contained a significantly higher number of vessels after 8 weeks.

### Assessing Heart Function

The assessment of a suitable stem cell therapy requires serial measurements of myocardial function, perfusion, viability and cell tracking.

Echocardiography is well established to measure myocardial function. Single photon emission computed tomography (SPECT) and positron emission tomography (PET) can also determine myocardial perfusion and metabolism. However, they are not able to resolve transmural analysis of the myocardium wail. In addition, dedicated set-ups are needed for small animal imaging, but these are not widely available.

Recently, magnetic resonance imaging (MRI) has emerged as a promising and recognized tool for cardiac imaging, as it will be further developed. MRI is now accepted as a gold standard for the assessment of the cardiac anatomy and volumes including the ejection fraction (EF) and cardiac output. Cine MRI provides a highly reproducible protocol to assess the myocardial contraction. Using special saturation pulses, the myocardium can be tagged far quantitative measurement of regional strains. MRI is also very sensitive to myocardial perfusion when first-pass contrast media study are performed. Finally, new MRI myocardial viability methods have been validated using contrast media as gadolinium chelates which induce a hyperenhancement of infarct myocardium. MRI assessment of viability correlates with PET for transmural infarct but appears to detect subendocardial infarction missed by PET.

As the MRI contrast and resolution between types of cells is limited, a new strategy has been developed to monitor in *vivo* specific groups of cells. Since the *in vivo* uptake of MRI contrast media can not be tailored to any type of cells, *ex-vivo* cells are typically labeled using paramagnetic or superparamagnetic iron oxide (SPIO) contrast media. After implantation, the labeled cells locally destroy the magnetic field homogeneity. This results on dark spots that can be detected using dedicated MRI sequences. However, the assessment is largely qualitative based on the presence or absence of the dark spots. A quantitative assessment of the contrast media and cell distribution in the tissue remains a significant and largely unresolved challenge.

### In vitro cell labeling

Two types of MR contrast agents are used clinically: gadolinium-analogues and iron oxide nanoparticles. These agents are particularly designed as blood-pool contrast agents which are impermeable to cells. Superparamagnetic nanoparticles of anionic y-Fe₂0₃ (iron oxide) label efficiently different types of cells due to the negative surfaces charges. These particles are thus particularly suited for cellular imaging *in vivo,* due to a near-cellular (i.e. 20-50 microns) resolution, long half-life and low local or systemic toxicity. However their uptake by cells still needs to be improved, for example using lipofection agents. A preferred magnetic labeling approach is based on the use of the FDA-approved SPIO formulation Ferridex, mixed with a transfection agent. A recent work by Hoehn *et al* used such a labeling to track GFP-expressing ESC (green fluorescent protein), by MRI upon implantation into an ischemic rat brain.

### Examples

### Example 1: cardiac Patches

Experiments were performed which establish the feasibility of growing mouse ESC, as well as differentiating ESC within embryoid bodies and neonatal cardiomyocytes into 3D-fibrin gels.

Ongoing studies on the differentiation of EPC show that the presence of vascular endothelial growth factor (VEGF) is preferred for their differentiation into mature endothelial cells. Indeed, EPC have the capability to create tube-like constructs in fibrin gels in presence of the epidermal growth factor (EGF). *In vivo* properties of the 3D cardiac patches were studied in order to investigate the fate of stem cells after implantation, their spatial distribution, their rate of migration *in situ* and the survival of the grafted cardiac patches.

The feasibility of rat heart imaging by MRI at 1.5 T are illustrated by the performance of serial in *vitro* MR imaging of labeled cells suspended in fibrin gels within spectrophotometric cuvettes or multi-well plates to develop a quantitative protocol to measure the iron content inside the cells. This MR protocol is applied to measure the time evolution of the iron concentration *in vivo* after cardiac patch engraftment.

The applicability of cell containing-3D fibrin gels into infarcted areas of a rat model of myocardial infarction in order to replace nonfunctional diseased cardiac tissue are evaluated, in particular by performing in *vivo* MRI monitoring of the 3D-labeled cardiac patches after heart implantation to define the bio-distribution of the stem cells in correlation to the cardiac function and infarct evolution.

### Example 2: ex vivo examination by immunohistochemical techniques to evaluate cell fate and biodistribution.

In preliminary tests the feasibility of fixing empty fibrin gels on the left ventricle of normal hearts was examined. Furthermore, in a first attempt to engraft gels containing undifferentiated ESC, cell survival and proliferation were observed for up to 2 weeks.

The 3-D gel systems used for manufacture of the cardiac patches are based on extracellular matrix proteins; the gel systems have the following properties:
- E-Modulus (elastic characteristics): 30-8OkPa
- Type of material: gel-like material with high water content, typically of 90 to 95%.

The 3D gel systems are designed so that different types of cells can be employed and combined within the gels:
- non human embryonic stem cells committed to a cardiac phenotype, and
- adult stem cells (such as endothelial progenitors cells), able to improve the neovascularization of the damaged tissue and/or to transdifferentiate to the cardiac phenotype.

### Example 3: isolation of MNCs and CD34+ cells from human umbilical cord blood (UCB)

UCB cell collection was approved by the ethical committee of the University Hospital Zurich. Typically, 50 mL of UCB could be collected from fresh placentas with umbilical cord unto Vacutainer tubes containing citrate as anticoagulant. The UCB was diluted with two volumes of Ca²⁺ and Mg²⁺ free Dulbecco PBS (D-PBS). Mononuclear cells (MNCs) were isolated by density gradient centrifugation with Biocoll (Oxid AG, Basel, Switzerland), then washed 3 x in D-PBS. Positive selection of CD34+ was performed by a magnetic bead separation method (MACS; Miltenyi Biotec, Gladbach, Germany), using the manufacturer's protocol.

### Example 4: culture of Endothelial Progenitor Cells (EPCs)

2 x 10⁴ MACS-selected CD 133+ cells were plated on 8-well glass culture dishes (Nunc Lab-Tek II Chamber slide system; VWR International AG, Dietikon, Switzerland) coated with 10 ng/mL human fibronectin (Bioreba, Basel, Switzerland) and 1% gelatin, or fibrin gel substrates. Cells were seeded in endothelial cell growth medium (EC) (C-22010; Cloritech, Palo Alto, CA; this medium initially contains 2% FBS and the additive C-3921 5) supplemented to 20% fetal calf serum (FCS). Cultures were grown at 37°C, 5% CO₂, in a humidified atmosphere. After 24 hr, the non-adherent cells were removed from adherent cells via careful replacement of medium with fresh EC medium.

### Example 5: Growth of EPCs on two-dimensional fibrin gels for cardiac implants

100 µL fibrin gel substrates were formed at the bottom glass of tissue culture chambers (Nunc Lab-Tek II Chamber slide system). Epidermal growth factor (EGF), or VEGF₁₆₅ were admixed to fibrin gel substrate at 150 ng/mL gel Control fibrin substrates were prepared with neither growth factor. 2 x iO⁴ MACS-selected CD133+ cells were seeded in 400 µL Clonthech EC medium atop the fibrin gel substrates formulated with growth factors. Assuming an even distribution of freely diffusing EGF or VEGF₁₆₅ between fibrin gel substrate and overlaying culture medium, this resulted in an initial concentration of 50 ng/mL EGF or VEGF₁₆₅ in the culture system. The culture medium was changed after 24 hr. A second change of medium after another 48 hr was critical for cell survival. Subsequent changes of EC culture medium were performed after 48 hr or 72 hr. In each change, 350 µL culture supernatant were removed and replaced with an equal volume of fresh EC medium. These changes of medium resulted in removal of EGF and VEGF and the concomitant decrease of EGF or VEGF₁₆₅ concentrations in the culture system. The concentrations of growth factors was calculated to be 21 ng/mL after the first change of medium, and 8.8 ng/mL, 3.7 ng/mL and 1.5 ng/mL after the second, third, and fourth change, respectively. After 14 days of cultivation a substantial part of the gel was proteolized. Therefore, in order to implant the gels on the top of the infarcted area, a 1% fibrin gel was added on top of the cells. After extraction of the gel from the well, these were implanted in the infarcted area and fixed with two sutures on the myocardium.

An analogous procedure was applied for non-human embryonic stem cells and other adult stem/progenitor cells used. For mouse ESC, stable clones were fist engineered with the CD63-EGFP marker gene to isolate green ESC-derived cardiomyocytes upon differentiation within embryoid bodies. For hESC, we used EFGP driven by EF1α promoter and an antibiotic cassette to obtain stable clones. 3D-fibrin gels containing undifferentiated ESC were monitored at day 0 and 4 days later. This allowed us to follow their fate *in vitro* within the gels (either alone or in combination with EPCs), as well as to identify them *in situ* after their engraftment. Gels containing embryoid bodies (EBs) formed by mouse ESC were preincubated for 6 days to allow cardiac differentiation. With time (up to 2 weeks), the EBs within the gels spread and cells migrated. Areas of beating cardiomyocytes were observed. Similarly, freshly dissociated and purified neonatal rat cardiomyocytes redifferentiate over time (up to 14 days) in the 3D gels and remain viable for up to 2 weeks in culture, forming a network of spontaneously beating cells.

### Example 6: implantation in a Rat model of myocardial infarction

Male Sprague-Dawley rats weighing 300-350 grams were initially anesthetized with 4-5% isofluorane in an induction chamber. Following the shaving and weighting, the rat was intubated with a 14-gauge catheter, tracheal ventilation was performed at 70 cycles/min with 2.5-3.0 mL tidal volume, room air supplemented with oxygen (Harvard Rodent Ventilator, Model 683, Harvard Apparatus Co, Inc.). 1.5-2% isofluorane was maintained for continuous anesthesia.

Three electrodes were positioned to record the electrocardiographic tracing (ECG) monitor. The respiration curve was also recorded during all procedure.

A left intercostal thoracotomy was performed under aseptic technique. The fourth intercostal space was opened carefully to avoid accidentally cutting any vessels including the internal mammary artery. The fourth and fifth ribs were separated with a small retractor (Harvard Apparatus, France) to explore the heart. The pericardium was removed, the left anterior descending artery and its branch was observed under surgical microscope. A 6-0 polypropylene snare was made passing through the epicardium layer around the origin of the artery between the left atrium and the right pulmonary outflow tract, tying the ligature permanently occluded the artery. After LAD ligature, the left ventricular anterior free wall becomes hypokenetic and clearer due to the cyanosis. The muscle layer and skin were closed with 3-O suture afterwards.

Before the rat woke up completely, extubation was performed and the rat was places in a recovery cage with a supply of oxygen for 30 to 60 minutes.

3D cardiac patches implantation was performed into two different conditions of the rats: with or without myocardial infarction. Echo study was 30 performed for left ventricular function evaluation in MI rats. week, 4 or 8 weeks after implantation, the rats were sacrificed and the histological and pathological studies were performed. To evaluate the transformation of left ventricular function, the echocardiograph study was performed to the rats with infarction the day before cells grafting and the day before sacrificing respectively.

Engraftments were performed on the normal hearts, immediately after coronary ligation, 1-week or 4 weeks after myocardial infarction. After 2 weeks, the immunohistochemistry staining revealed a gel full of proliferating ESC, positive for the proliferating cell nuclear antigen (PCNA) and the GFP marker, as shown in figure 6.

Rats were anesthetized, and under sterile technique, the chest was re-opened. The infarcted area was identified visually on the basis of surface scar and wall motion abnormality. 3D patches are applied and fixed onto the of the left ventricular anterior free wall. Control animals received empty 3D patches.

To prevent rejection and assess the effect of an immunosuppression treatment on stem cell fate, groups of rats received immunosuppression agent cyclosporin A delivered continuously via an osmotic minipump (ALZA Corporation). Alzet mini-osmotic pumps were filled with cyclosporin A (CsA) (Sandimmune, Novartis 50mg/1ml), and was kept overnight at 37°C in PBS before implantation. The CsA release was adjusted at 2.5 µl/hour or 10 µl/hour and pump were designed for a 7-or 28-days release. The administrated dosage of CsA was calculated as 6-9 mg/Kg/day. After hair shaving and skin cleaning at the site for incision, a hemostat was inserted into the incision to spread the subcutaneous tissue and create a prompt pocket for the pump. The filled pump was implanted subcutaneously and the wound closed with suture. All procedure was performed under sterile circumstances.

### Example 7: Evaluation of the left ventricular function by echocardiography

For the evaluation of left ventricular function, transthoracic echocardiogram was performed on the rats after myocardial infarction week or 4 weeks right before implantation (baseline echocardiogram), and 1 week or 4 weeks after implantation, before the sacrifice of the animals. Rats were anesthetized with 4-5% isofluorane in an induction chamber. The chest was shaved, the rats were placed in dorsal decubitus position and intubated for continuous ventilation. 1-2% isofluorane was continuously supplied via a mask. 3 electrodes were adhered to their paws to record the electrocardiographic tracing simultaneously with the cardiac image identifying the phase of a cardiac cycle. Echocardiograms were performed with a commercially available echocardiography system equipped with 7.5 MHz phased-array transducer (Philips-Hewlett-Packard). The transducer was positioned on the left anterior side of the chest. At first, longitudinal images of the heart were obtained, including the left ventricle, atrium, the mitral valve and the aorta, followed by the cross-sectional images from the plane of the base to the left ventricular apical region. M-mode tracings were obtained at the level below the tip of the mitral valve leaflets at the level of the papillary muscles. All of two-dimensional images, M-mode tracings and Doppler curves were recorded on videotape for later analysis. We calculated the fractional shortening (FS) as a measure of systolic function, according to the M-mode tracing from the cross-sectional view:
maximal LV end-diastolic diameter (LVEDD, at the time of maximal cavity dimension), minimal LV end-systolic diameter (LVESD, at the time of maximum anterior motion of the posterior wall), FS (%) = {(LVEDD-LVESD) / LVEDD} x 100. All measurements were averaged for 3 consecutive cardiac cycles.

Our experiments established the feasibility of growing mouse ESC, as well differentiating ESC within embryoid bodies and neonatal cardiomyocytes into 3D-fibrin gel systems. Figure 3 shows a pictoral representation of the different multi-well systems used to produce the 3D-fibrin gels. Furthermore, the ongoing studies on the differentiation of EPC show the importance of the presence of vascular endothelial growth factor (VEGF) in the differentiation of the stem cells into mature endothelial cells. Indeed, EPC have the capability to create tube-like constructs in fibrin gels in presence of the epidermal growth factor (EGF).

The feasibility of fixing empty 3D fibrin gel systems on the left ventricle of normal hearts. Furthermore, in an attempt to engraft a 3D fibrin gel system containing undifferentiated ESC, cell survival and proliferation for up to 2 weeks were observed.

Cell therapy of heart failure is presently performed using autologous adult stem cells (from bone marrow or skeletal muscle compartment) has entered recently clinical phase 1 and 2 trials. However, cell injection via a syringe is highly ineffective and results in the loss of more than 95% of the cells. The cardiac patches allow a better survival and the correct insertion of the appropriate cells where the tissue need to be regenerated. Furthermore, the vascularization of these tissues is of main importance for the repopulation of the damaged heart and thus the patient survival. Another crucial advantage is the fact that the gels are formed by extracellular matrix proteins present in normal tissues and biodegradable by endogenous and cell-released proteases.

### Bibliography

- Frank JA, Zywicke H, Jardan EK, Mitchell J, Lewis BK, Miller B, Bryant LH, Jr., Bulte JW. Magnetic intracellular labeling of mammalian cells by combining (FDA-approved) superparamagnetic iron oxide MR contrast agents and commonly used transfection agents. A cad Radiol. 2002;9:S484-7.
- Nugent HM, Edebman BR. Tissue engineering therapy for cardiovascular disease. Circ Res. 2003;92: 1068-78.
- Shimizu T, Yamato M, Kikuchi A, Okano T. Cell sheet engineering for myocardial tissue reconstruction. Biomaterials. 2003;24:23 09-16.
- Zimmermann WH, Fink C, Kralisch D, Remmers 13, Weil J, Eschenhagen T. Three-dimensional engineered heart tissue from neonatal rat cardiac myocytes. Biotechnol Bioeng. 2000;68:106-14.
- Ai-Saadi N, Nagel E, Grass M, Bomstedt A, Schnackenburg B, Klein C, Kiimek W, Oswaid H, Fieck E. Noninvasive detection of myocardial ischemia from perfusion reserve based on cardiavascular magnetic resonance. Circulation. 2000;101 :1379-83.
- Anderson DJ, Gage FH, Weissman IL. Can stem cells cross lineage boundaries? Nat Med. 2001;7:393-5.
- Asahara T, Murohara T, Sullivan A, Silver M, van der Zee R, Li T, Witzenbichler B, Schatteman G, Isner JM. Isolation of putative progenitor endothelial cells for angiogenesis. Science. 1997;275:964-7.
- Brown AN, Kim BS, Alsberg E, Mooney DJ. Combining chondrocytes and smooth muscle cells to engineer hybrid soft tissue constructs. Tissue Eng. 2000;6:297-305
- Burg KJ, Holder WD, Jr., Culberson CR, Beiler RI, Greene KG, Laebsack AB, Roland WD, Eiselt P, Maoney DJ, Halberstadt CR. Comparative study of seeding methods for three-dimensional polymeric scaffoids. J Biomed Mater Res. 2000;52:576.
- Chenite A, Chaput C, Wang D, Combes C, Buschmann MD, Hoemann CD, Leroux JC, Atkinsan BL, Binette F, Selmani A. Novel injectable neutral solutions of chitosan form biodegradable gels in situ. Biomaterials. 30 2000;21:2155-61
- Croisille P, Maare CC, Judd RM, Lima JA, Arai M, McVeigh BR, Becker LC, Zerhouni EA. Differentiation of viable and nonviable myocardium by the use of three-dimensional tagged MRI in 2-day-Old reperfused canine infarcts. Circulation. 1999;99:284-291.
- Dar A, Shachar M, Leor J, Cohen S. Optimization of cardiac cell seeding mand distribution in 3D porous alginate scaffolds. Biotechnol Bioeng. 2002;80:305-1 2.
- Dowell JD, Rubart M, Pasuniarthi KB, Soonpaa MH, Field LJ. Myocyte and myogenic stem cell transplantation in the heart. Cardiovasc Res. 20 2003;58:336-50.
- Duranti F, Salti G, Bovani B, Calandra M, Rasati ML. Injectable hyaluronic acid gel for soft tissue augmentation. A clinical and histological study. Dermatol Surg. I 998;24: 1317-25.
- Edeiberg JM, Tang L, Hattori K, Lyden D, Rafii 5. Young adult bone marrow-derived endothelial precursor cells restore aging-impaired cardiac angiogenic function. Circ Res. 2002;90:E89-93.
- Eschenhagen T, Didie M, Heubach J, Ravens 13, Zimmermann WH. Cardiac tissue engineering. Transpl Jmmunol. 2002;9:3 15-21.
- Gray MO, Lang CS, Kalinyak JE, Li HT, Karliner JS. Angiotensin II stimulates cardiac myocyte hypertrophy via paracrine release of TGF-beta 1 AND endothelin-1 from fibroblasts. Cardiovasc Res. 1998 ;40:352-63.
- Hall H, Baechi T, Hubbell JA. Molecular properties of fibrin-based matrices for promotion of angiogenesis in vitro. Microvasc Res. 2001 ;62 :315-26.
- Hoehn M, Kustermann E, Blunk J, Wiedermann D, Trapp T, Wecker 5, Focking M, Arnold H, Hescheler J, Fleischmann BK, Schwindt W, Buhrle C. Monitoring of implanted stem cell migration in vivo: a highly resolved in vivo magnetic resonance imaging investigation of experimental stroke in rat. Proc Nati Acad Sci USA. 2002;99: 16267-72.
- Hoffmann R, Lethen H, Marwick T, Arnese M, Fioretti P, Pingitore A, Picana E, Buck T, Erbel R, Flachskampf FA, Hanrath P. Analysis of interinstitutional observer agreement in interpretation of dobutamine stress echocardiagrams. J Am Coll Cardiol. 1996;27:330-3 36.
- Holder WD, Jr., Gruber HE, Moore AL, Culbersan CR, Anderson W, Burg KJ, Moaney DJ. Cellular ingrowth and thickness changes in poly-L-lactide and polyglycolide matrices implanted subcutaneously in the rat. J Biomed Mater Res. 1998;41:412-21.
- Jones JM, Thomson JA. Human embryonic stem cell technology. Semin Reprod Med. 2000;18:219-23.
- Kalka C, Masuda H, Takahashi T, Kalka-Moll WM, Silver M, Kearney M, Li T, Isner JM, Asahara T. Transplantation of ex vivo expanded endothelial progenitor cells for therapeutic neovascularization. Proc Natl Acad Sci USA. 2000;97:3422-7.
- Kim RI, Wu E, Rafael A, Chen BL, Parker MA, Simonetti O, Kiocke FJ, Banow RO, Judd RM. The use of contrast-enhanced magnetic resonance imaging to identify reversible myocardial dysfunction. N Engl J Med. 2000;343: 1445-53.
- Klein C, Nekoila SG, Bengel FM, Mamose M, Samnier A, Haas F, Schnackenburg B, Delius W, Mudra H, Wolfram D, Schwaiger M. Assessment of myocardial viability with contrast-enhanced magnetic resonance imaging: comparison with positron emission tomography. Circulation. 2002; 105:162-7.
- Klug MG, Soonpaa MH, Koh GY, Field LJ. Genetically selected cardiomyocytes from differentiating embronic stem cells from stable intracardiac grafts. J Clin Jnvest. 1996;98:216-24.
- Koh GY, Soonpaa MH, Kbug MG, Pride HP, Cooper BJ, Zipes DP, Field LJ. Stable fetal cardiomyocyte grafts in the hearts of dystrophic mice and dogs. J Clin Jnvest. 1995;96:2034-42.
- Koh GY, Soonpaa MH, Klug MG, Fieid LJ. Strategies for myocardial repair. J Interv Cardiol. 1995;8:387-93.
- Kraitchman DL, Heldman AW, Atalar E, Amado LC, Martin BJ, Pittenger MF, Hare JM, Bulte JW. In vivo magnetic resonance imaging of mesenchymal stem cells in myocardial infarction. Circulation. 2003;107:2290-3.
- Lear J, Abouiafia-Etzion S, Dar A, Shapiro L, Barbash IM, Battler A, Granot Y, Cahen S. Bioengineered cardiac grafts: A new approach to repair the infarcted myocardium Circulation. 2000; 102:11156-61.
- Long CS, Henrich CJ, Simpson PC. A growth factor for cardiac myocytes is produced by cardiac nonmyocytes. Cell Regul. 1991;2:1081-95.
- Menasche P. Myoblast-based cell transplantation. Heart Fail Rev. 2003;8:221-7.
- Meyer N, Jaconi M, Landapoulou A, Fort P, Puceat M. A fluorescent reporter gene as a marker for ventricular specification in ES-derived cardiac cells. FEBS Lett. 2000;478:151-8.
- Murohara T, Ikeda H, Duan J, Shintani S, Sasaki K, Eguchi H, Onitsuka I, Matsui K, Imaizumi T. Transplanted cord blood-derived endothelial precursor cells augment postnatal neovascularization. J Clin Jnvest. 2000; 105:1527-36.
- Odorico JS, Kaufh-ian DS, Thamsan JA. Multilineage differentiation from human embryonic stem cell lines. Stem Cells. 2001 ;19:193-204.
- Passier R, Mummery C. Origin and use ofembryonic and adult stem cells in differentiation and tissue repair. Cardiovasc Res. 2003;58:324-35.
- Pasumarthi KB, Fieid LJ. Cardiomyocyte enrichment in differentiating cell cultures: strategies and applications. Methods Mol Biol. 2002; 185:157-68.
- Pautler RG, Fraser SE. The year(s) of the contrast agent - micro-MRI in the new millennium. Curr Opin Immunol. 2003;15:385-92.
- Schense JC, Bloch J, Aebischer P, Hubbeil JA. Enzymatic incorporation of bioactive peptides into fibrin matrices enhances neurite extension. Nat Biotechnol. 2000;1 8:415-9.
- Schense JC, Hubbell JA. Crass-lirìking exogenaus bifunctional peptides into fibrin gels with factor XIIIa. Bioconjug Chem. 1999;10:75-81.
- Schmeisser A, Strasser RH. Phenotypic overlap between hematopoietic cells with suggested angioblastic potential and vascular endothelial cells. J Hematother Stem Cell Res. 2002;1 1:69-79.
- Shah AM, Grocott-Mason RM, Pepper CB, Mebazaa A, Henderson AH, Lewis MJ, Paulus WJ. The cardiac endothelium: cardioactive mediators. Prog Cardiovasc Dis. 1996;39:263-84
- Shimizu T, Yamato M, Isoi Y, Akutsu T, Setomaru T, Abe K, Kikuchi A, Umezu M, Qkana T. Fabrication of pulsatile cardiac tissue grafts using a navel 3-dimensional cell sheet manipulation technique and temperature- responsive celi culture surfaces. Circ Res. 2002;90:e40.
- Takahashi I, Kalka C, Masuda H, Chen D, Silver M, Kearney M, Magner M, Isner JM, Asahara T. Ischemia- and cytaoine-induced mobilization of bone marrow-derived endothelial progenitor cells for neovascularization. Nat Med. 1999;5:434-8.
- Takeshita 5, Zheng LP, Bragi E, Keamey M, Pu LQ, Bunting 5, Ferrara N, Symes JF, Isner JM. Therapeutic angiogenesis. A single intraarterial bolus of vascular endothelial growth factor augments revascularization in a rabbit ischemic hind limb model. J Clin Invest. 1994;93 :662-70.
- Tateishi-Yuyama E, Matsubara H, Murohara T, Ikeda 13, Shintani S, Masaki H, Amano K, Kishimoto Y, Yoshimoto K, Akashi H, Shiniada K, Iwasaka T, Imaizumi T. Therapeutic angiogenesis for patients with limb ischaemia by autologous transplantation of bone-marrow cells: a pilot study and a randomised controlled trial. Lancet. 2002;360:427-35.
- Tharnson JA, Itskovitz-Eldar J, Shapiro SS, Waknitz MA, Swiergiel JJ, Marshaii VS, Jones JM. Embryonic stem cell lines derived from human blastocysts. Science 1998;282:1 145-7.
- Wagers AJ, Sherwood RI, Christensen JL, Weissman IL. Little evidence for developmental plasticity of adult hematopoietic stem cells. Science,2002;297:2256-9.
- Weissman IL. Stem cells: units of development, units of regeneration, and units in evolution. Cell 2000;100:157-68.
- Ye Q, Zund G, Benedikt P, Jockenhaevel S, Hoerstrup SP, Sakyama 5, Hubbeil JA, Turina M. Fibrin gel as a three dimensional matrix in cardiovascular tissue engineering. Eur J Cardiohorac Surg. 2000; 17:587-91
- Zimmermann WH, Didie M, Wasmeier GH, Nixdarff 13, Hess A, Melnychenko I, Bay O, Neuhuber WL, Weyand M, Eschenhagen T. Cardiac grafting of engineered heart tissue in syngenic rats. Circulation. 2002;106:1151-7.
- Zimmermann WH, Eschenhagen T. Cardiac tissue engineering for replacement therapy. Heart Fail Rev. 2003;8:259-69.
- Zisch AH, Schenk U, Schense JC, Sakiyama-Elbert SE, Hubbeli JA. Covalently conjugated VEGF--fibrin matrices for endothelialization. J Control Release. 2001;72:101.

## Claims

1. Composition for use in treating heart failure comprising: a biodegradable gel-based matrix made of fibrin, at least one growth factor or cytokine incorporated in the matrix, and cardiogenic stem cells wherein said cardiogenic stem cells do not comprise human embryonic stem cells, **characterized in that** it is in the form of patch.

2. Composition for use according to claim 1 wherein the biodegradable gel-based matrix has an elasticity expressed in E-Modulus of 30-80 kPa and a water content of 90-95%.

3. Composition for use according to claim 1 wherein the growth factors are chosen in the group consisting of: vascular endothelial growth factor (VEGF), epidermal growth factor (EGF), plateled-derived growth factor (PDGF), transforming growth factor beta (TGFβ), insulin growth factor 1 (IGF1), placental growth factor (PLGF), keratinocyte-derived growth factor (KDGF).

4. Composition for use according to claim 1 wherein the cytokines are chosen from the group consisting of interleukin 6 (IL-6) family, soluble c-kit ligand (s-kitL) and cardiotrophin-1.

5. Composition for use according to claim 4 wherein the cytokines of IL-6 family are: IL-6, leukemia inhibitory factor (LIF).

6. Use of the composition of claims 1 to 5 for the preparation of a medicament to treat heart failure.

7. Method for the preparation of the composition of claims 1-5 comprising the following steps:
a) forming a gel matrix made of fibrin of claim 1;
b) admixing to the gel matrix made from step a) active agents of claims 3 to 5;
c) seeding on the gel matrix made of fibrin from step b) non human stem cells;
d) cultivating cells from step c) for up to 14 days;
e) steps a-d are eventually repeated sequentially.

8. The Composition for use according to claims 1-5 to be administered in the form of a gel based matrix performed in vitro just before implantation or to be formed upon injections into the heart.

## Patentansprüche

1. Zusammensetzung zur Verwendung bei der Behandlung von Herzinsuffizienz, umfassend:
eine biologisch abbaubare Gel-basierte Matrix aus Fibrin, mindestens einen Wachstumsfaktor oder Cytokin, der bzw. das in die Matrix integriert ist, und kardiogene Stammzellen,
wobei die kardiogenen Stammzellen keine menschlichen embryonalen Stammzellen umfassen, **dadurch gekennzeichnet, dass** dieselbe die Form eines Patches hat.

2. Zusammensetzung zur Verwendung gemäß Anspruch 1, wobei die biologisch abbaubare Gel-basierte Matrix eine in E-Modul ausgedrückte Elastizität von 30 - 80 kPa und einen Wassergehalt von 90 - 95% hat.

3. Zusammensetzung zur Verwendung gemäß Anspruch 1, wobei die Wachstumsfaktoren aus der Gruppe ausgewählt sind, die besteht aus: vaskulärem endothelialem Wachstumsfaktor (VEGF), epidermalem Wachstumsfaktor (EGF), Plättchen-deriviertem Wachstumsfaktor (PDGF), transformierendem Wachstumsfaktor Beta (TGFβ), Insulinwachstumsfaktor-1 (IGF1), plazentarem Wachstumsfaktor (PLGF), Keratinozyten-deriviertem Wachstumsfaktor (KDGF).

4. Zusammensetzung zur Verwendung gemäß Anspruch 1, wobei die Cytokine aus der Gruppe ausgewählt sind, die aus der Interleukin-6- (IL-6-) Familie, löslichem c-kit-Ligand (s-kitL) und Kardiotrophin-1 besteht.

5. Zusammensetzung zur Verwendung gemäß Anspruch 4, wobei die Cytokine der IL-6-Familie IL-6, Leukämie-inhibierender Faktor (LIF) sind.

6. Verwendung der Zusammensetzung gemäß den Ansprüchen 2 bis 5 zum Präparieren eines Medikamentes zur Behandlung von Herzinsuffizienz.

7. Verfahren zum Präparieren der Zusammensetzung gemäß den Ansprüchen 1 - 5, das die folgenden Schritte umfasst:
a) Bilden einer Gelmatrix aus Fibrin gemäß Anspruch 1;
b) Beimischen von Wirkstoffen gemäß den Ansprüchen 3 bis 5 zu der in Schritt a) hergestellten Gelmatrix;
c) Aussäen von nichtmenschlichen Stammzellen auf der Gelmatrix aus Fibrin aus Schritt b);
d) Kultivieren von Zellen aus Schritt c) für bis zu 14 Tage;
e) die Schritte a - d werden gegebenenfalls sequentiell wiederholt.

8. Zusammensetzung zur Verwendung gemäß den Ansprüchen 1 - 5 zur Verabreichung in Form einer Gel-basierten Matrix, die in vitro kurz vor der Implantation durchgeführt wird, oder zur Bildung bei Injektionen ins Herz.

## Revendications

1. Composition pour l'utilisation dans le traitement de l'insuffisance cardiaque comprenant : une matrice à base de gel biodégradable constituée de fibrine, au moins un facteur de croissance ou cytokine incorporé dans la matrice et des cellules souches cardiogéniques, dans laquelle lesdites cellules souches cardiogéniques ne comprennent pas de cellules souches embryonnaires humaines, **caractérisée en ce qu'**elle est sous la forme de pansement.

2. Composition pour l'utilisation selon la revendication 1, dans laquelle la matrice à base de gel biodégradable a une élasticité exprimée en module d'élasticité de 30-80 kPa et une teneur en eau de 90-95%.

3. Composition pour l'utilisation selon la revendication 1, dans laquelle les facteurs de croissance sont sélectionnés dans le groupe comprenant :
facteur de croissance endothélial vasculaire (VEGF), facteur de croissance épidermique (EGF), facteur de croissance dérivé des plaquettes (PDGF), facteur de croissance transformant bêta (TGFβ), facteur de croissance de l'insuline 1 (IGF1), facteur de croissance placentaire (PLGF), facteur de croissance dérivé des kératinocytes (KDGF).

4. Composition pour l'utilisation selon la revendication 1, dans laquelle les cytokines sont sélectionnées dans le groupe comprenant : la famille de l'interleukine 6 (IL-6), un ligand c-kit soluble (skitL) et la cardiotrophine-1.

5. Composition pour l'utilisation selon la revendication 4, dans laquelle les cytokines de la famille IL-6 sont : facteur inhibiteur de leucémie (LIF) IL-6.

6. Utilisation de la composition selon les revendications 1 à 5 pour la préparation d'un médicament pour traiter l'insuffisance cardiaque.

7. Procédé pour la préparation de la composition selon les revendications 1 à 5, comprenant les étapes suivantes :
a) la formation d'une matrice de gel constituée de fibrine selon la revendication 1 ;
b) le mélange de la matrice de gel réalisée à partir de l'étape a) avec des agents réactifs selon les revendications 3 à 5 ;
c) l'ensemencement sur la matrice de gel constituée de fibrine provenant de l'étape b) de cellules souches non humaines ;
d) la culture de cellules provenant de l'étape c) jusqu'à 14 jours ;
e) les étapes a-d sont éventuellement répétées de manière séquentielle.

8. Composition pour l'utilisation selon les revendications 1 à 5 destinée à être administrée sous la forme d'une matrice à base de gel réalisée in vitro juste avant l'implantation ou destinée à être formée lors d'injections dans le coeur.
